# EUROPÄISCHE PATENTSCHRIFT

(11) **EP 1 625 551 B1**
(45) Veröffentlichungstag und Bekanntmachung des Hinweises auf die Patenterteilung: **21.07.2010**
(21) Anmeldenummer: 04728347.8
(22) Anmeldetag: 20.04.2004
(51) Int. Cl.: G01N 29/04, G07D 7/08, G07D 7/18

(54) **VERFAHREN UND VORRICHTUNG ZUR BESTIMMUNG DER LAPPIGKEIT VON BLATTGUT MITTELS ULTRASCHALL**
METHOD AND DEVICE FOR DETERMINING THE LIMPNESS OF SHEET MATERIAL BY MEANS OF ULTRASOUND
PROCEDE ET DISPOSITIF POUR DETERMINER LA MOLLESSE DE FEUILLES AU MOYEN D'ULTRASONS

(30) Priorität: 22.04.2003 DE 10318104
(43) Veröffentlichungstag der Anmeldung: 15.02.2006
(73) Patentinhaber: Giesecke & Devrient GmbH, 81677 München (DE)
(72) Erfinder: PRADEL, Helmut, 80804 München (DE)
(74) Vertreter: Klunker . Schmitt-Nilson . Hirsch
(86) Internationale Anmeldenummer: PCT/EP2004/004176
(87) Internationale Veröffentlichungsnummer: WO 2004/095380

(56) Entgegenhaltungen:
- EP-A- 0 470 808
- DE-A- 10 029 442
- US-A- 5 922 959
- US-A- 6 026 681
- US-A- 6 115 127
- US-A1- 2003 025 512

## Beschreibung

Die Erfindung betrifft ein Verfahren und eine Vorrichtung zur Bestimmung der Lappigkeit von Blattgut, insbesondere von Banknoten, bei denen das Blattgut mit Schallwellen bestrahlt, die von dem bestrahlten Blattgut ausgehenden Schallwellen gemessen und die Bestimmung der Lappigkeit des Blättguts aufgrund der gemessenen Schallwellen erfolgt.

Ein solches System ist beispielsweise aus der US 5,922,959 bekannt. Hierbei wird der von einer beschallten Banknote reflektierte Schallanteil gemessen. Je weniger Schall von der geprüften Banknote reflektiert wird, um so größer ist der Grad der Lappigkeit der Banknote. Weiterhin ist beschrieben, daß alternativ auch der Grad der Transmission von Schallwellen bestimmt werden kann.

In DE 100 29 442 A1 ist ein Verfahren zur Bestimmung von Strukturinhomogenitäten in Blattgut, insbesondere Knitterfalten oder Rissen in Banknoten, beschrieben, bei dem das Blattgut mit Ultraschall beaufschlagt und der durch das Blatt transmittierte oder am Blattgut reflektierte Ultraschall gemessen wird. Es wird dann ein für den transmittierten bzw. reflektierten Ultraschall charakteristischer Wert erzeugt, aus dem ein Maß für die Strukturinhomogenitäten im Blattgut bestimmt wird.

Diese bekannte Vorgehensweise hat allerdings den Nachteil, daß sie nur unzureichend den Grad der Lappigkeit von Banknoten bestimmen kann. Andere Einflußgrößen wie der Verschmutzungsgrad der Banknote, Falten oder Knitter führen ebenfalls zu einer veränderten Absorption von Schall, so daß die bei dem bekannten System gewünschte Information über die Lappigkeit des Banknotenpapiers nicht eindeutig durch die bekannten Verfahren gewonnen werden kann.

Davon ausgehend ist es die Aufgabe der vorliegenden Erfindung, ein Verfahren und eine Vorrichtung zur Bestimmung der Lappigkeit von Blattgut bereitzustellen, welche eine höhere Genauigkeit aufweisen.

Diese Aufgabe wird durch das Verfahren nach Anspruch 1 und die Vorrichtung nach Anspruch 10 gelöst. Die weiteren Ansprüche beschreiben bevorzugte Ausgestaltungen.

Ein wesentlicher Gedanke der vorliegenden Erfindung beruht somit darauf, daß sowohl das vom Blattgut reflektierter, als auch transmittierter Schall gemessen und ein mathematisches Verhältnis der reflektierten und transmittierten Schallwellen gebildet wird, um die Lappigkeit zu bestimmen. Diese Verhältnisbildung kann zum Beispiel in der Bildung einer Differenz oder eines Quotienten der Intensitäten der gemessenen Signale bestehen. Durch die Berücksichtigung beider Anteile bei einer Messung besteht die Möglichkeit, Einflüsse anderer Eigenschaften des Blattguts, wie z.B. von dessen Zustand, Verschmutzungsgrad, Flächengewicht etc., zu verringern. Diese Messung wird dabei besonders bevorzugt an einer gemeinsamen Stelle des Blattgutes erfolgen, da es bei Messung an verschiedenen Stellen, z.B. aufgrund eines unterschiedlichen Verschmutzungsgrades, zu Ungenauigkeiten bei der Lappigkeitsbestimmung kommen kann. Zudem kann nicht nur lokal, sondern zusätzlich oder alternativ auch ganzflächig bestrahlt und ausgewertet werden.

Neben der vorstehend genannten Variante der Verhältnisbildung von transmittierter und reflektierter Schallwelle gibt es noch weitere Vorgehensweisen, die auch unabhängig voneinander und der vorstehend genannten erfindungsgemäßen Lösung eingesetzt werden können.

So kann zur Kompensation der genannten Blattguteigenschaften beispielsweise auch vorgesehen sein, daß neben der Messung der Lappigkeit noch eine weitere Messung zur Bestimmung dieser Blattguteigenschaft, wie des Nennwerts, des Flächengewichts, oder des Verschmutzungsgrades der bestrahlten Stelle des Blattgutes durchgeführt und diese Blattguteigenschaft bei der Bestimmung der Lappigkeit berücksichtigt wird. Dies bedeutet z. B., daß der Lappigkeitsmessung vor- oder alternativ auch nachgeschaltet eine separate Verschmutzungsmessung erfolgt, wobei das erzielte Maß zum Verschinutzungsgrad bei der Bildung eines Referenzwertes berücksichtigt werden kann, mit dem die Ergebnisse der akustischen Lappigkeitsmessung verglichen werden, um über den Grad der Lappigkeit zu entscheiden. Die Verschmutzungsmessung kann dabei anhand der bekannten Verfahren, z. B. nach DE 27 52 412 A1, DE 29 32 962 A1 oder DE 100 05 514 A1 erfolgen.

Die Genauigkeit der Auswertung der Lappigkeit kann vorzugsweise auch dadurch erhöht werden, daß der bekannte oder zuvor per Messung bestimmte Nennwert der jeweiligen Banknote berücksichtigt wird. Dies kann z.B. durch Wahl unterschiedlicher Vergleichswerte für unterschiedliche Nennwerte erfolgen.

Eine weitere unabhängige Idee der vorliegenden Erfindung besteht darin, daß nicht bloß die Intensität und damit der Anteil der reflektierten beziehungsweise transmittierten Schallwellen gemessen, sondern das Frequenzspektrum der gemessenen Schallwellen bestimmt wird. Lappige Banknoten sollten z.B. ein anderes Frequenzspektrum aufweisen, als nichtlappige Banknoten. Hierbei kann eine Messung zumindest eines Frequenzbandes und/ oder mehrerer diskreter Frequenzen erfolgen. Zudem kann eine Auswertung der Intensität und/oder Phase der unterschiedlichen Frequenzen, durchgeführt werden. Insbesondere bei dieser Messung wird bevorzugt eine Impulsanregung und/oder eine Anregung mit mehreren Frequenzen erfolgen.

Noch eine weitere unabhängige Idee der vorliegenden Erfindung besteht darin, die Laufzeit von Schallwellen im Blattgut selbst zu messen. So kann beispielsweise eine Banknote an einer bestimmten Stelle des Papiers zu einem bestimmten Zeitpunkt mit einem Schallimpuls bestrahlt und z. B. mit Hilfe eines optischen Interferometers die nachfolgende Auslenkung der Banknote an einer davon beabstandeten Stelle zeitaufgelöst gemessen werden, um ein Maß über die Ausbreitung der Schallwelle in dem Banknotenpapier zu erhalten. Die Laufzeit und/ oder die Größe der Auslenkung wird üblicherweise von dem Grad der Lappigkeit der Banknote abhängt. Die Laufzeit kann dabei bevorzugt bei mehreren Frequenzen gemessen und die Messung bei mehreren Frequenzen ausgewertet werden.

Eine weitere unabhängige Idee der vorliegenden Erfindung besteht darin, die Anregung des Blattguts und/ oder die Messung der vom Blattgut ausgehenden Schallwellen berührungsbehaftet durchzuführen. So kann vorzugsweise ein Ultraschallwandler zum Aussenden und/oder Empfangen von Schallwellen so angeordnet sein, daß er teilweise oder vollständig mit einem zu prüfenden Blatt in Kontakt ist bzw. kommt. Hierdurch wird im Vergleich zu einer kontaktlosen Anregung/Messung der Wirkungsgrad erhöht und störende Einflüsse durch die Übertragung in der Luft verringert.

Die vorliegende Erfindung wird nachfolgend anhand der beigefügten Zeichnungen näher erläutert. Dabei zeigt
- Fig. 1: eine schematische seitliche Ansicht auf eine Prüfvorrichtung zur Be- stimmung der Lappigkeit nach einem ersten Ausführungsbeispiel; und
- Fig. 2: eine schematische seitliche Ansicht auf eine Vorrichtung zur Bestim- mung der Lappigkeit nach einem zweiten Ausführungsbeispiel.

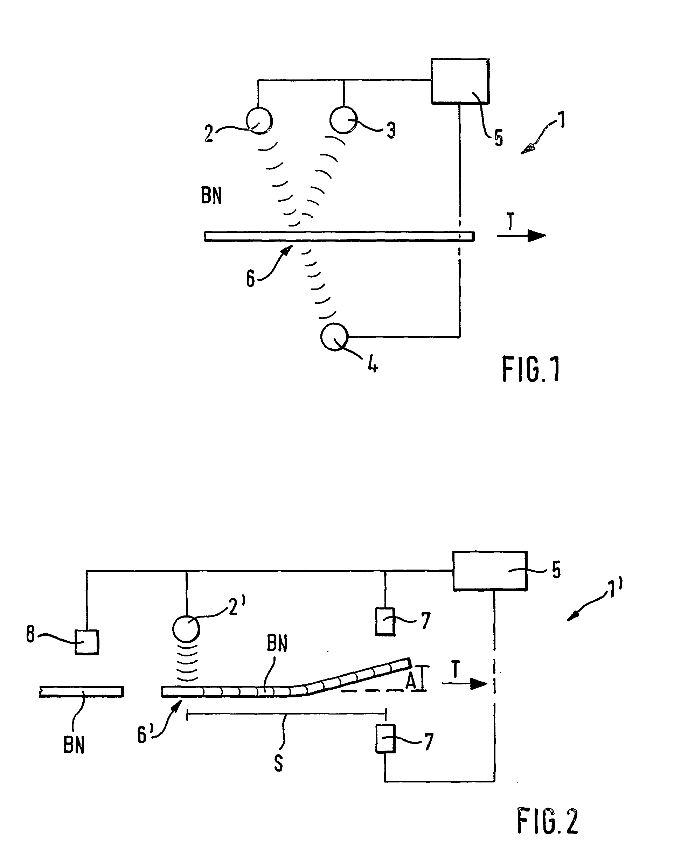

Fig. 1 zeigt ein erstes Beispiel für eine Prüfvorrichtung 1 zur Bestimmung der Lappigkeit von Banknoten BN. Die Prüfvorrichtung 1 kann dabei in an sich bekannter Weise in einer Vorrichtung zur Bearbeitung von Banknoten BN integriert sein, bei der Banknoten im vereinzelten Zustand an der Prüfeinrichtung 1 in Richtung T vorbeitransportiert werden. Die Vorrichtung 1 weist dabei im Speziellen eine Schallquelle 2 zur Bestrahlung des Blattguts mit Schallwellen und eine Meßeinrichtung zum Messen der von dem beschallten Blattgut reflektierten und transmittierten Schallwellen auf. Im Speziellen weist die Meßeinrichtung einen Reflexionssensor 3 zur Messung des von der Banknote BN reflektierten Schallanteils und einen Transmissionssensor 4 zur Messung des durch die Banknote BN transmittierten Schallanteils auf.

Zudem umfaßt die Vorrichtung 1 noch eine Auswertungseinrichtung 5, die über Signalleitungen mit der Schallquelle 2 und den Sensoren 3,4 verbunden ist und zur Auswertung der Signale dieser Komponenten ausgelegt ist, um die Lappigkeit der geprüften Banknote BN zu bestimmen. Obwohl nicht darauf beschränkt, wird die Schallquelle 2 vorzugsweise Ultraschallwellen aussenden. Die Frequenzen werden dabei z. B. in einem Bereich von 100 - 400 kHz liegen. Die Schallquelle 2 und die Sensoren 3,4 sind dabei so angeordnet, und haben eine solche Abstrahl- bzw. Meßcharakteristik, daß sie die von derselben Stelle 6 ausgehenden Schallanteile messen können. In der Auswertungseinrichtung 5 werden die für eine Stelle 6 gemessenen Schallanteile dann verglichen und dabei z. B. in ein Differenzverhältnis oder ein Quotientenverhältnis der Schallintensitäten gesetzt, welches mit einem oder mehreren vorbestimmten Referenzwerten verglichen wird, um den Grad der Lappigkeit zu bestimmen. Dieses Maß für die Lappigkeit der Banknote BN kann dabei Einflussgrößen wie das Flächengewicht oder den Verschmutzungsgrad der Banknote BN teilweise kompensieren, die sich ansonsten störend auf die Messung auswirken würden. Die Stelle 6, an der beschallt und gemessen wird, kann sich nur über einen Teilbereich der Banknotenfläche erstrecken. Es ist allerdings auch möglich, daß die Banknote großflächig bestrahlt und auch großflächig gemessen wird und/oder die Meßwerte von mehreren Stellen 6 einer einzelne Banknote BN aufgenommen und z.B. gemittelt ausgewertet werden. Zudem ist denkbar, daß die Banknote BN vor der Auswertung der Lappigkeitsmessung auf Fehler, wie z.B. Risse, Löcher oder dergleichen untersucht und die Auswertung der Lappigkeitsmessung an solchen fehlerhaften Stellen nicht durchgeführt wird.

Die Fig. 2 zeigt ein zweites Ausführungsbeispiel der vorliegenden Erfindung. Die Vorrichtung 1' umfaßt dabei wiederum eine Schallquelle 2' zur akustischen Beschallung einer zur prüfenden Banknote BN und umfaßt ebenfalls die nicht dargestellten Sensoren 3 und 4 zur Erfassung von reflektiertem bzw. transmittiertem Schall umfassen.

Allerdings ist diese Variante dadurch besonders gekennzeichnet, daß sie zusätzlich oder alternativ zur Messung der Laufzeit von Schallwellen in der Banknote dient. Hierzu weist die Vorrichtung 1' vorzugsweise zwei auf gegenüberliegenden Seiten befindliche optische Sensoren 7 auf. Diese Sensoren 7 können z. B. optische Interferometer 7 sein, welche eine Auslenkung A der Banknote BN aufgrund einer Schallanregung bestimmen können. Im Speziellen wird nun eine zu prüfende Banknote BN zu einer gegebenen Zeit an einer bestimmten Stelle 6' lokal mit einem kurzen Schallimpuls durch die Schallquelle 2' beaufschlagt und zeitaufgelöst danach durch die optischen Interferometer 7 die Auslenkung A der Banknote BN in dem Bereich zwischen den beiden gegenüberliegenden Interferometern 7 bestimmt, der beabstandet von der Stelle 6' ist. Die gemessenen Signale werden in der Auswertungseinrichtung 5 dahingehend ausgewertet, wie lang die Laufzeit des Schalls für die Strecke S zwischen dem Beaufschlagungspunkt des Schalls 6' und dem Meßbereich der beiden Interferometer 7 ist. Das Maß der Laufzeit wird dabei alleine oder in Kombination mit den Messungen der reflektierten bzw. transmittierten Schallanteile zur Bestimmung der Lappigkeit verwendet. Es wird angenommen, daß die Laufzeit in dem Banknotenpapier um so größer ist, je größer die Lappigkeit und damit die Zerstörung der inneren Struktur des Banknotenpapieres ist. Alternativ kann auch mit anderen Meßverfahren als optischer Inferometrie die Laufzeit des Schalls durch Erfassung der Auslenkung A der Banknoten BN an voneinander beabstandeten Stellen bestimmt werden.

Als weiteres Merkmal weist die Prüfeinrichtung 1' nach Fig. 2. optional noch einen Sensor zur Messung anderer Banknoteneigenschaften, wie z.B. einen Verschmutzungssensor 8 auf, der z. B. gemäß DE 29 32 962 A1 ausgestaltet sein kann, um den Verschmutzungsgrad einer Banknote BN zu bestimmen. Der Verschmutzungssensor 8 kann z. B. dazu ausgelegt sein, Licht im unsichtbaren Spektralbereich zu empfangen, um daraus den Grad der Verschmutzung der Banknote BN festzustellen. Der Verschnutzungssenor 8 ist dabei bevorzugt dem Lappigkeitssensor im Transport Richtung T der einzelnen Banknoten BN vorgeschaltet. Die Bestimmung des Verschmutzungsgrades mit dem Verschmutzungssensor 8 wird dabei vorzugsweise an derselben Stelle erfolgen, an der auch nachfolgend die Lappigkeit bestimmt wird. Weil eine verschmutzte Banknote BN üblicherweise mehr Schall absorbieren sollte als eine nicht verschmutzte Banknote BN, unabhängig vom Lappigkeitsgrad, können die mittels des Verschmutzungssensors 8 bestimmten Ergebnisse zum Grad der Verschmutzung einer zu prüfenden Banknote BN bei der nachfolgenden Auswertung in der Auswertungseinrichtung 5 zur Lappigkeitsbestimmung berücksichtigt werden. Zusätzlich oder alternativ können anstelle der Verschmutzung auch der Nennwert oder das Flächengewicht der Banknote BN vorhergehend bestimmt und bei der Auswertung der Lappigkeit berücksichtigt werden.

Diese Variante mit vorgeschaltetem Sensor zur Messung anderer Banknoteneigenschaften, wie des Verschmutzungssensors 8, ist insbesondere auch bei anderen akustischen Lappigkeitsmessungen von Vorteil, bei denen z.B. nicht das Verhältnis von transmittierter und reflektierter Schallintensität, sondern lediglich in bekannter Weise nur der reflektierte oder alternativ der transmittierte Schallanteil bestimmt wird. In diesem Fall wird z.B. zumindest der eine Referenzwert, mit dem die gemessene Schallintensität zur Bestimmung der Lappigkeit verglichen wird, in Abhängigkeit vom gemessenen Verschmutzungsgrad unterschiedlich gewählt.

Neben den vorstehend genannten Varianten sind noch weitere Ausgestaltungen denkbar. So können die Sensoren 3,4, welche zur Messung von reflektierten bzw. transmittierten Schallwellen dienen, vorzugsweise als Breitbandmikrofone ausgestaltet sein, die dazu in der Lage sind, ein breites Frequenzspektrum der gemessenen Schallsignale zu erfassen, so daß dieses in der Auswertungseinrichtung 5 ausgewertet werden kann. Die Schallsensoren 3, 4 können dabei bevorzugterweise Breitbandmikrofone mit einer Aufnahmecharakteristik im Bereich von 0,1 kHz bis 200 kHz haben.

Insesondere in diesem Fall wird auch eine Breitbandanregung mittels Beschallung mit einem Schallimpuls oder einer mechanischen Anregung durch Ab- bzw. Umlenkung der Banknote erfolgen. So kann die BN beispielsweise an einer Kufe im Transportweg abgelenkt werden und die entstehenden Geräusche breitbandig gemessen werden.

Die Auswertung des Frequenzspektrums von Schallwellen, die von der zu prüfenden Banknote BN ausgehen, haben dabei charakteristische Formen für lappige im Vergleich zu nichtlappigen Banknoten. Es sei betont, daß diese Idee der Auswertung des Frequenzspektrums und nicht nur der Intensität der gemessenen Schallwellen auch unabhängig von der akustischen Anregung verwendet werden kann. Sie ist beispielsweise auch bei einer bekannten mechanischen Anregung von Banknoten und anschließender Messung der von der mechanisch angeregten Banknote ausgehenden Schallwellen einsetzbar.

Vorzugsweise werden die Schallquellen und/oder die Meßeinrichtungen in Kontakt mit dem zu messenden Blattgut sein, um eine verbesserte Signalübertragung mit geringeren Störeinflüssen zu erreichen.

Die vorliegende Erfindung in den unterschiedlichen Varianten erlaubt folglich eine einfache und sichere Bestimmung der Lappigkeit von Banknoten.

## Patentansprüche

1. Verfahren zur Bestimmung der Lappigkeit von Blattgut (BN), insbesondere Banknoten (BN), mit den Schritten:
- Bestrahlen des Blattguts (BN) mit Schallwellen,
- Messen der Schallwellen, die von dem bestrahlten Blattgut (BN) ausgehen,
- Bestimmen der Lappigkeit des Blattguts (BN) aufgrund der gemessenen Schallwellen,
**dadurch gekennzeichnet, daß**
sowohl vom Blattgut (BN) reflektierte, als auch transmittierte Schallwellen gemessen werden, und ein mathematisches Verhältnis der reflektierten und transmittierten Schallwellen gebildet wird, um die Lappigkeit zu bestimmen.

2. Verfahren nach Anspruch 1, **dadurch gekennzeichnet, daß** die reflektierten, als auch die transmittierten Schallwellen von einer gemeinsamen Stelle (6) des Blattguts (BN) gemessen werden.

3. Verfahren nach einem der vorherigen Ansprüche, **dadurch gekennzeichnet, daß** ein Maß für eine andere Eigenschaft des Blattguts als die Lappigkeit, wie z.B. der Nennwert des Blattguts, das Flächengewicht und/ oder der Verschmutzungsgrad des Blattguts (BN) bestimmt und bei der Bestimmung der Lappigkeit berücksichtigt wird.

4. Verfahren nach einem der vorherigen Ansprüche, **dadurch gekennzeichnet, daß** ein Maß für die das Blattgut (BN) bestrahlenden Schallwellen gemessen und bei der Verhältnisbildung zur Bestimmung der Lappigkeit berücksichtigt wird.

5. Verfahren nach einem der vorherigen Ansprüche, **dadurch gekennzeichnet, daß** das Frequenzspektrum der Schallwellen gemessen und bei der Bestimmung der Lappigkeit berücksichtigt wird.

6. Verfahren nach einem der vorherigen Ansprüche, **dadurch gekennzeichnet, daß** die Laufzeit von Schallwellen im Blattgut (BN) gemessen und bei der Bestimmung der Lappigkeit berücksichtigt wird.

7. Verfahren nach einem der vorherigen Ansprüche, **dadurch gekennzeichnet, daß** zur Bestimmung der Laufzeit von Schallwellen im Blattgut (BN) eine Messung, insbesondere eine optische Messung, der Auslenkung (A) des Blattguts (BN) durchgeführt wird.

8. Verfahren nach einem der vorherigen Ansprüche, **dadurch gekennzeichnet, daß** fehlerhafte Bereiche des Blattguts bestimmt und diese Bereiche bei der Bestimmung der Lappigkeit nicht berücksichtigt werden.

9. Verfahren nach einem der vorherigen Ansprüche, **dadurch gekennzeichnet, daß** die Anregung des Blattguts und/oder die Messung der vom Blattgut ausgehenden Schallwellen berührungsbehaftet durchgeführt wird.

10. Vorrichtung (1,1') zur Bestimmung der Lappigkeit von Blattgut (BN), insbesondere Banknoten (BN), mit
- einer Schallquelle (2, 2') zum Bestrahlen des Blattguts (BN) mit Schallwellen,
- einer Meßeinrichtung (3, 4) zum Messen der Schallwellen, die von dem bestrahlten Blattgut (BN) ausgehen,
- einer Auswertungseinrichtung (5) zur Bestimmung der Lappigkeit des Blattguts (BN) aufgrund der durch die Meßeinrichtung (3, 4) erfaßten Schallwellen,
**dadurch gekennzeichnet, daß**
die Meßeinrichtung (3, 4) sowohl einen Reflexionssensor (3) zur Messung der vom Blattgut (BN) reflektierten Schallwellen, als auch einen Transmissionssensor (4) zur Messung der durch das Blattgut (BN) transmittierten Schallwellen aufweist, und die Auswertungseinrichtung (5) ausgelegt ist, ein mathematisches Verhältnis der gemessenen reflektierten und transmittierten Schallwellen zu bilden, um die Lappigkeit zu bestimmen.

11. Vorrichtung nach Anspruch 10, **dadurch gekennzeichnet, daß** die Auswertungseinrichtung (5) ausgelegt ist, ein mathematisches Verhältnis der von einer gemeinsamen Stelle (6, 6') des Blattguts (BN) ausgehenden reflektierten und transmittierten Schallwellen zu bilden, um die Lappigkeit zu bestimmen.

12. Vorrichtung nach zumindest einem der Ansprüche 10 oder 11, **dadurch gekennzeichnet, daß** die Meßeinrichtung (3, 4) ein Breitbandmikrophon (3, 4) aufweist, um das Frequenzspektrum der erfaßten Schallwellen zu bestimmen.

13. Vorrichtung nach zumindest einem der Ansprüche 10 bis 12, **dadurch gekennzeichnet, daß** die Meßeinrichtung (3,4,7) eine Einheit (7) zur Bestimmung der Laufzeit von Schallwellen im Blattgut (BN) aufweist.

14. Vorrichtung nach zumindest einem der Ansprüche 10 bis 13, **dadurch gekennzeichnet, daß** die Meßeinrichtung (3, 4, 7) eine Einheit (7) zur Bestimmung einer anderen Eigenschaft des Blattguts als die Lappigkeit, wie z.B. den Nennwert des Blattguts, des Flächengewichts und/ oder des Verschmutzungsgrades des Blattguts (BN) aufweist.

15. Vorrichtung nach zumindest einem der Ansprüche 10 bis 14, **dadurch gekennzeichnet, daß** die Schallquelle (2, 2') und/oder die Meßeinrichtung (3, 4) in Kontakt mit dem zu messenden Blattgut (BN) ist.

## Claims

1. A method for determining the limpness of sheet material (BN), in particular bank notes (BN), having the steps of:
- irradiating the sheet material (BN) with sound waves,
- measuring the sound waves emanating from the irradiated sheet material (BN),
- determining the limpness of the sheet material (BN) on the basis of the measured sound waves,
**characterized in that**
both sound waves reflected by the sheet material (BN) and those transmitted thereby are measured, and a mathematical relation of reflected and transmitted sound waves is formed in order to determine the limpness.

2. The method according to claim 1, **characterized in that** both the reflected and the transmitted sound waves are measured from a common place (6) on the sheet material (BN).

3. The method according to either of the above claims, **characterized in that** a measure of a property of the sheet material other than limpness, such as e.g. the denomination of the sheet material, the weight per unit area and/or the degree of soiling of the sheet material (BN), is determined and taken into consideration upon determination of limpness.

4. The method according to any of the above claims, **characterized in that** a measure of the sound waves irradiating the sheet material (BN) is measured and taken into consideration upon formation of the relation for determining limpness.

5. The method according to any of the above claims, **characterized in that** the frequency spectrum of the sound waves is measured and taken into consideration upon determination of limpness.

6. The method according to any of the above claims, **characterized in that** the travel time of sound waves within the sheet material (BN) is measured and taken into consideration upon determination of limpness.

7. The method according to any of the above claims, **characterized in that** for determining the travel time of sound waves within the sheet material (BN), a measure- ' ment, in particular an optical measurement, of the deflection (A) of the sheet material (BN) is carried out.

8. The method according to any of the above claims, **characterized in that** defective areas of the sheet material are determined and said areas are not taken into consideration upon determination of limpness.

9. The method according to any of the above claims, **characterized in that** the excitation of the sheet material and/or the measurement of the sound waves emanating from the sheet material is carried out in contacting fashion.

10. An apparatus (1, 1') for determining the limpness of sheet material (BN), in particular bank notes, having
- a sound source (2, 2') for irradiating the sheet material (BN) with sound waves,
- a measuring device (3, 4) for measuring the sound waves emanating from the irradiated sheet material (BN),
- an evaluation device (5) for determining the limpness of the sheet material (BN) on the basis of the sound waves captured by the measuring device (3, 4),
**characterized in that**
the measuring device (3, 4) has both a reflection sensor (3) for measuring the sound waves reflected by the sheet material (BN) and a transmission sensor (4) for measuring the sound waves transmitted through the sheet material (BN), and the evaluation device (5) is designed to form a mathematical relation of the measured reflected and transmitted sound waves in order to determine limpness.

11. The apparatus according to claim 10, **characterized in that** the evaluation device (5) is designed to form a mathematical relation of the reflected and transmitted sound waves emanating from a common place (6, 6') on the sheet material (BN) in order to determine limpness.

12. The apparatus according to at least one of claims 10 to 11, **characterized in that** the measuring device (3, 4) has a broadband microphone (3, 4) in order to determine the frequency spectrum of the captured sound waves.

13. The apparatus according to at least one of claims 10 to 12, **characterized in that** the measuring device (3, 4, 7) has a unit (7) for determining the travel time of sound waves within the sheet material (BN).

14. The apparatus according to at least one of claims 10 to 13, **characterized in that** the measuring device (3, 4, 7) has a unit (7) for determining a property of the sheet material other than limpness, such as e.g. the denomination of the sheet material, the weight per unit area and/or the degree of soiling of the sheet material (BN).

15. The apparatus according to at least one of claims 10 to 14, **characterized in that** the sound source (2, 2') and/or the measuring device (3, 4) is in contact with the sheet material (BN) to be measured.

## Revendications

1. Procédé de détermination de la flaccidité de produit en feuilles (BN), notamment billets de banque (BN), comprenant les étapes :
- irradiation du produit en feuilles (BN) avec des ondes acoustiques,
- mesure des ondes acoustiques qui proviennent du produit en feuilles (BN) irradié,
- détermination de la flaccidité du produit en feuilles (BN) sur la base des ondes acoustiques mesurées,
**caractérisé en ce que**
des ondes acoustiques tant réfléchies que transmises par le produit en feuilles (BN) sont mesurées et une relation mathématique des ondes acoustiques réfléchies par rapport aux ondes acoustiques transmises est établie pour déterminer la flaccidité.

2. Procédé selon la revendication 1, **caractérisé en ce que** tant les ondes acoustiques réfléchies.que les ondes acoustiques transmises sont mesurées à un endroit (6) commun du produit en feuilles (BN).

3. Procédé selon une des revendications précédentes, **caractérisé en ce qu'**une mesure est déterminée pour une propriété du produit en feuilles autre que la flaccidité, à savoir p.ex. pour la valeur nominale du produit en feuilles, le grammage et/ou le degré de souillure du produit en feuilles (BN), et est prise en compte dans la détermination de la flaccidité.

4. Procédé selon une des revendications précédentes, **caractérisé en ce qu'**une grandeur est mesurée pour les ondes acoustiques irradiant le produit en feuilles (BN) et est prise en compte dans l'établissement de la relation pour la détermination de la flaccidité.

5. Procédé selon une des revendications précédentes, **caractérisé en ce que** le spectre de fréquence des ondes acoustiques est mesuré et pris en compte dans la détermination de la flaccidité.

6. Procédé selon une des revendications précédentes, **caractérisé en ce que** le temps de transit d'ondes acoustiques dans le produit en feuilles (BN) est mesuré et pris en compte dans la détermination de la flaccidité.

7. Procédé selon une des revendications précédentes, **caractérisé en ce que**, pour la détermination du temps de transit d'ondes acoustiques dans le produit en feuilles (BN), une mesure, notamment une mesure optique, de la déflection (A) du produit en feuilles (BN) est effectuée.

8. Procédé selon une des revendications précédentes, **caractérisé en ce que** des zones défectueuses du produit en feuilles (BN) sont déterminées et **en ce que** ces zones ne sont pas prises en compte dans la détermination de la flaccidité.

9. Procédé selon une des revendications précédentes, **caractérisé en ce que** l'excitation du produit en feuilles (BN) et/ou la mesure des ondes acoustiques provenant du produit en feuilles (BN) est effectuée avec contact.

10. Dispositif (1, 1') de détermination de la flaccidité de produit en feuilles (BN), notamment billets de banque (BN), comprenant
- une source acoustique (2, 2') pour irradier du produit en feuilles (BN) avec des ondes acoustiques,
- un équipement de mesure (3, 4) pour mesurer des ondes acoustiques provenant du produit en feuilles (BN) irradié,
- un équipement d'évaluation (5) pour la détermination de la flaccidité de produit en feuilles (BN) sur la base des ondes acoustiques saisies par l'équipement de mesure (3, 4),
**caractérisé en ce que**
l'équipement de mesure (3, 4) comporte tant un capteur de réflexion (3) pour la mesure des ondes acoustiques réfléchies par le produit en feuilles (BN) qu'un capteur de transmission (4) pour la mesure des ondes acoustiques transmises par le produit en feuilles (BN), et **en ce que** l'équipement d'évaluation (5) est conçu pour établir quant aux ondes acoustiques mesurées une relation mathématique entre les ondes réfléchies et les ondes transmises, afin de déterminer la flaccidité.

11. Dispositif selon la revendication 10, **caractérisé en ce que** l'équipement d'évaluation (5) est conçu pour établir quant aux ondes acoustiques provenant d'un endroit commun (6, 6') du produit en feuilles (BN) une relation mathématique entre les ondes réfléchies et les ondes transmises, afin de déterminer la flaccidité.

12. Dispositif selon au moins une des revendications 10 ou 11, **caractérisé en ce que** l'équipement de mesure (3, 4) comporte un microphone large bande (3, 4) afin de déterminer le spectre de fréquence des ondes acoustiques saisies.

13. Dispositif selon au moins une des revendications de 10 à 12, **caractérisé en ce que** l'équipement de mesure (3, 4, 7) comporte une unité (7) pour la détermination du temps de transit d'ondes acoustiques dans le produit en feuilles (BN).

14. Dispositif selon au moins une des revendications de 10 à 13, **caractérisé en ce que** l'équipement de mesure (3, 4, 7) comporte une unité (7) pour la détermination d'une propriété du produit en feuilles (BN) autre que la flaccidité, à savoir p.ex. pour la valeur nominale du produit en feuilles (BN), le grammage et/ou le degré de souillure du produit en feuilles (BN).

15. Dispositif selon au moins une des revendications de 10 à 14, **caractérisé en ce que** la source acoustique (2, 2') et/ou l'équipement de mesure (3, 4) est en contact avec le produit en feuilles (BN) devant être mesurés.
